(19) Europäisches Patentamt
European Patent Office
Office européen des brevets

(11) **EP 3 659 536 A1**

(12) **EUROPEAN PATENT APPLICATION**

(43) Date of publication:
**03.06.2020 Bulletin 2020/23**

(51) Int Cl.:
**A61B 18/12** (2006.01)   **A61B 18/14** (2006.01)
**A61B 18/00** (2006.01)

(21) Application number: **19209947.1**

(22) Date of filing: **19.11.2019**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **20.11.2018 US 201816196255
01.03.2019 US 201916290819**

(71) Applicant: **Biosense Webster (Israel) Ltd.
Yokneam, 2066717 (IL)**

(72) Inventors:
• **GOVARI, Assaf**
**2066717 Yokneam (IL)**
• **ALTMANN, Andres Claudio**
**2066717 Yokneam (IL)**
• **OZERI, Ella**
**2066717 Yokneam (IL)**

(74) Representative: **Small, Gary James
Carpmaels & Ransford LLP
One Southampton Row
London WC1B 5HA (GB)**

(54) **IRRIGATION CONTROL DURING ABLATION**

(57)      In one embodiment, an irrigated ablation system includes a probe to be inserted into a chamber of a heart, the probe including an electrode, a temperature sensor to provide a temperature signal indicative of a temperature of a myocardium, and an irrigation channel through which to irrigate the myocardium, a pump to pump an irrigation fluid into the irrigation channel, an RF signal generator to generate RF power to be applied by the electrode to ablate the myocardium, and a controller to receive the temperature signal, calculate a rate of change of the temperature over time based on the temperature signal, calculate an irrigation rate with which to irrigate the myocardium via the irrigation channel based at least on the calculated rate of change of the temperature, and provide an irrigation signal to the pump to irrigate the myocardium with the irrigation fluid at the calculated irrigation rate.

Fig. 1

**Description**

RELATED APPLICATION INFORMATION

The present application is a Continuation-in-part of US Patent Application S/N 16/196,255 of Govari, et al., filed November 20, 2018.

FIELD OF THE INVENTION

The present invention generally relates to an ablative medical device, and specifically to control of parameters used during ablation performed by the device.

BACKGROUND

Ablation of tissue, such as ablation performed by injecting radiofrequency (RF) power into the tissue, is a well-known procedure that is used, for example, to correct defects in the heart. Typically, in these cases the ablation is used to inactivate selected groups of cells in the myocardium, so that they no longer transfer an electropotential wave in the myocardium.

US Published Patent Application Serial Number 2011/0022041 of Frank, et al., describes a system for ablating tissue comprising an electrode configured for use to deliver RF power to ablate the tissue, and a heat flow sensor configured to provide a measurement of heat flow from the electrode to blood or irrigation fluid.

US Patent 5,304,214 to DeFord, et al., describes a catheter, system, and method for selectively ablating prostatic tissue about the prostatic urethra. The catheter includes an elongated member having distal, proximal, and intermediate portions, the intermediate portion being shaped and sized for intimate contact with the prostatic urethra. The distal and proximal portions of the catheter include fixation and cooling balloons having an annular recess therein for positioning the internal and external sphincters therein and for maintaining the longitudinal position of the catheter in the prostatic urethra. A thermally conducted, heat-emitting element is positioned in the intermediate portion for producing a thermally conductive heat distribution to ablate the prostatic tissue. The catheter also includes irrigation and aspiration passageways therein for communicating with the interior of the distal and proximal cooling balloons. A circulating pump of the ablation system circulates coolant through the balloons to maintain the temperature of the sphincters below an injurious temperature. Sensors are positioned about the heat-emitting element as well as in the cooling balloons for supplying information to the controller of the system. The controller in response to the temperature information and the energy supplied to the heat-emitting element controls the supply of energy to the catheter as well as the pump circulating the coolant.

US Published Patent Application Serial Number 2008/0275440 of Kratoska, et al., describes a method of providing feedback regarding the outcome of ablation therapy.

US Published Patent Application Serial Number 2011/0077639 of Brannan, et al., describes a microwave ablation system includes a generator operable to output energy and an ablation probe coupled to the generator that delivers the energy to a tissue region. The ablation system also includes a controller operable to control the generator and at least one sensor coupled to the ablation probe and the controller that detects an operating parameter of the ablation probe. The controller performs a system check by ramping up an energy output of the generator from a low energy level to a high energy level and monitors an output from the sensor at predetermined intervals of time during the system check to determine an abnormal state. The controller controls the generator to cease the energy output when the controller determines an abnormal state.

SUMMARY

There is provided in accordance with an embodiment of the present disclosure, an irrigated ablation system including a probe being configured to be inserted into a chamber of a heart, the probe including an electrode configured to apply radiofrequency (RF) power to a myocardium in the chamber so as to ablate the myocardium, a temperature sensor configured to provide a temperature signal which is indicative of a temperature of the myocardium at a plurality of different times, and an irrigation channel through which to irrigate the myocardium, a pump to pump an irrigation fluid into the irrigation channel, an RF signal generator configured to generate the RF power to be applied by the electrode to ablate the myocardium, and a controller configured to receive the temperature signal from the temperature sensor, calculate a rate of change of the temperature over time based on the temperature signal, calculate an irrigation rate with which to irrigate the myocardium via the irrigation channel with the irrigation fluid based at least on the calculated rate of change of the temperature, and provide an irrigation signal to the pump to irrigate the myocardium with the irrigation fluid at the calculated irrigation rate.

Further in accordance with an embodiment of the present disclosure the controller is configured to calculate the irrigation rate based both on the calculated rate of change of the temperature and on a temperature difference, which is equal to a current temperature measured by the temperature sensor less a preset target temperature.

Still further in accordance with an embodiment of the present disclosure the controller is configured to calculate the irrigation rate based on a function that yields a higher irrigation rate based on a higher rate of change of temperature.

Additionally, in accordance with an embodiment of the present disclosure the function is configured to yield a higher irrigation rate based on a higher value of the temperature difference.

Moreover, in accordance with an embodiment of the present disclosure the controller is configured to calculate the irrigation rate based on the calculated rate of change of the temperature, the temperature difference, a rate of change of the RF power, and a RF power difference, which is equal to a difference between a current value of the RF power and a preset target RF power.

There is provided in accordance with another embodiment of the present disclosure, an irrigated ablation method including generating radiofrequency (RF) power to be applied by an electrode of a probe to ablate a myocardium in a chamber of a heart, applying the RF power to the myocardium so as to ablate the myocardium, providing a temperature signal which is indicative of a temperature of the myocardium at a plurality of different times, pumping an irrigation fluid into an irrigation channel through which to irrigate the myocardium, receiving the temperature signal, calculating a rate of change of the temperature over time based on the temperature signal, calculating an irrigation rate with which to irrigate the myocardium via the irrigation channel with the irrigation fluid based at least on the calculated rate of change of the temperature, and providing an irrigation signal to a pump to irrigate the myocardium with the irrigation fluid at the calculated irrigation rate.

Further in accordance with an embodiment of the present disclosure the calculating the irrigation rate includes calculating the irrigation rate based both on the calculated rate of change of the temperature and on a temperature difference, which is equal to a current temperature measured by the temperature sensor less a preset target temperature.

Still further in accordance with an embodiment of the present disclosure the irrigation rate is calculated based on a function that yields a higher irrigation rate based on a higher rate of change of temperature.

Additionally, in accordance with an embodiment of the present disclosure the function is configured to yield a higher irrigation rate based on a higher value of the temperature difference.

Moreover, in accordance with an embodiment of the present disclosure the calculating the irrigation rate includes calculating the irrigation rate based on the calculated rate of change of the temperature, the temperature difference, a rate of change of the RF power, and a RF power difference, which is equal to a difference between a current value of the RF power and a preset target RF power.

There is also provided in accordance with still another embodiment of the present disclosure a software product, including a non-transient computer-readable medium in which program instructions are stored, which instructions, when read by a central processing unit (CPU), cause the CPU to receive a temperature signal which is indicative of a temperature of a myocardium of a chamber of a heart at a plurality of different times, calculate a rate of change of the temperature over time based on the temperature signal, calculate an irrigation rate with which to irrigate the myocardium via an irrigation channel with an irrigation fluid based at least on the calculated rate of change of the temperature, and provide an irrigation signal to a pump to irrigate the myocardium with the irrigation fluid at the calculated irrigation rate.

BRIEF DESCRIPTION OF THE DRAWINGS

The present invention will be understood from the following detailed description, taken in conjunction with the drawings in which:

Fig. 1 is a partly pictorial, partly block diagram view of an ablation apparatus constructed and operative according to an embodiment of the present invention;

Fig. 2 is a schematic illustration of a distal end of a probe used in the apparatus of Fig. 1 according to an embodiment of the present invention;

Fig. 3 is a flow chart showing exemplary steps in a method of operation of the ablation apparatus of Fig. 1 according to an embodiment of the present invention;

Fig. 4 is a first flow chart showing exemplary steps comprised in an algorithm for use by the apparatus of Fig. 1;

Fig. 5 is a second flow chart showing exemplary steps comprised in the algorithm used by the apparatus of Fig. 1;

Fig. 6 illustrates graphically the operation of a pump of the apparatus while the flowcharts of Figs. 4 and 5 are operative, according to an embodiment of the present invention;

Fig. 7 is a first flowchart of steps of an alternative algorithm used by the apparatus, according to an embodiment of the present invention;

Fig. 8 is a second flowchart of steps of the alternative algorithm, according to an embodiment of the present invention; and

Fig. 9 illustrates graphically the operation of the pump of the apparatus while the flowcharts of Figs. 7 and 8 are operative, according to an embodiment of the present invention.

DESCRIPTION OF EXAMPLE EMBODIMENTS

OVERVIEW

During an ablation procedure the ablative power injected into cells needs to be well regulated, since if too little ablative energy is absorbed by the cells they may only partly inactivate, while if too much ablative energy is absorbed it may cause excessive injury to the heart, which can be life-threatening. Another consideration for the power injected is the overall time for any given ablation procedure. Physicians typically prefer to keep the time to a minimum, so that in order to inject sufficient energy, the power injected during this time should be high. Thus, a goal for ablative power delivery is that the power level should be as close as possible to a target power, subject to not causing excessive trauma.

Tissue irrigation is necessary during ablation of the myocardium, to prevent problems such as tissue charring, or cavitation (referred to as "steam-pops") occurring during the ablation. Therefore, in addition to the goal of the power level being as close as possible to a target power, the temperature of the myocardium tissue should remain as close as possible to a target temperature. A more stable temperature and power generally leads to better ablation results and a higher quality lesion.

Legacy ablation systems typically provide irrigation at one of two rates - a low irrigation rate which, inter alia, may be used to maintain irrigation channels such as to prevent clogging of the channels, and a high rate, which is used to prevent the temperature-related problems referred to above. However, the high rate may lead to the tissue being overcooled, and in this case ablation power must be delivered for a longer-than-optimal time to correctly ablate the tissue.

US Patent Application Publication 2018/0263689 and entitled "Simultaneous control of power and irrigation during ablation", which is incorporated herein by reference, describes a system to reduce the longer-than-optimal time delivery of legacy ablation systems by pulsing the irrigation rate between the low and high rates in a controlled manner. The pulsatory irrigation rate is smoothed, by tubing used to supply the irrigation fluid, so that the irrigation rate at the tissue is substantially constant. In addition, by varying the frequency at which high-rate pulses are applied, the smoothed irrigation rate may be varied in a substantially continuous manner between the low rate and the high rate.

The goals of power level and temperature regulation are further enhanced in embodiments of the present invention, which provides an apparatus that regulates the temperature by calculating an irrigation rate as a function of a rate of change of temperature of the myocardium tissue. This approach provides a faster reaction to temperature changes and thereby helps ensure that temperature is restored quickly to the target temperature.

The apparatus comprises a probe configured to be inserted into a chamber of a heart. The probe includes an electrode configured to apply radiofrequency (RF) power to the myocardium in the chamber so as to ablate the myocardium. The probe also includes a temperature sensor to provide a temperature signal which is indicative of a temperature of the myocardium at a plurality of different times, and an irrigation channel through which to irrigate the myocardium. The apparatus also includes an RF signal generator configured to generate the RF power to be applied by the electrode to ablate the myocardium, and a pump to pump an irrigation fluid into the irrigation channel. In accordance with some embodiments of the present invention the pump is a variable rate pump. In accordance with other embodiments of the present invention a variable irrigation rate may be provided using the method described in US Patent Application Publication 2018/0263689.

The apparatus also includes a controller to receive the temperature signal from the temperature sensor and calculate a rate of change of the temperature over time based on the temperature signal. The controller is configured to calculate an irrigation rate with which to irrigate the myocardium via the irrigation channel with the irrigation fluid based at least on the calculated rate of change of the temperature. In some embodiments, the controller is configured to calculate the irrigation rate based both on the calculated rate of change of the temperature and on a temperature difference, which is equal to a current temperature of the myocardium measured by the temperature sensor less a preset target temperature. The controller is also configured to provide an irrigation signal to the pump to irrigate the myocardium with the irrigation fluid at the calculated irrigation rate.

Documents incorporated by reference herein are to be considered an integral part of the application except that, to the extent that any terms are defined in these incorporated documents in a manner that conflicts with definitions made explicitly or implicitly in the present specification, only the definitions in the present specification should be considered.

SYSTEM DESCRIPTION

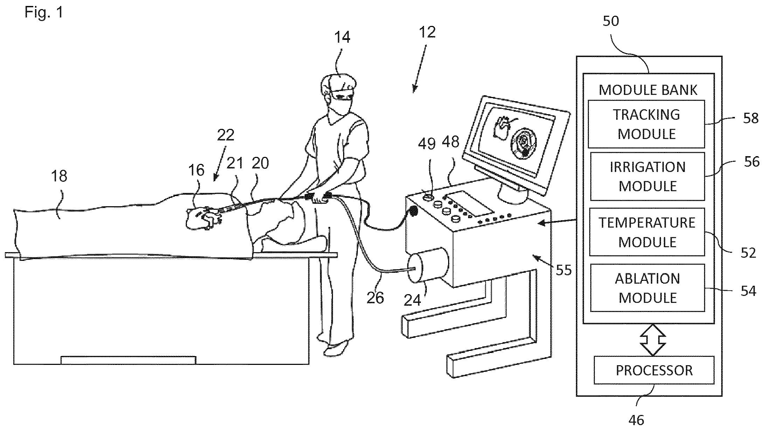

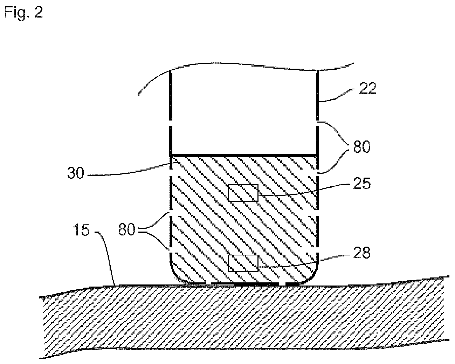

Reference is now made to Figs. 1 and 2. Fig. 1 is a partly pictorial, partly block diagram, view of an ablation apparatus 12 constructed and operative according to an embodiment of the present invention. Fig. 2 is a schematic illustration of a distal end 22 of a probe 20 used in the apparatus 12 of Fig. 1 constructed and operative according to an embodiment of the present invention. The probe 20 is configured to be inserted into a chamber of a heart. The procedure is performed by a medical professional 14, and, by way of example, the procedure in the description hereinbelow is assumed to comprise ablation of a portion 15 of a myocardium 16 of the heart of a human patient 18. However, it will be understood that embodiments of the present invention are not just applicable to this specific ablation procedure, and may include substantially any ablation procedure on biological tissue or on non-biological material.

In order to perform the ablation, the medical professional 14 inserts the probe 20 into a sheath 21 that has been pre-positioned in a lumen of the human patient 18. The sheath 21 is positioned so that the distal end 22 of the probe 20 may enter the heart of the patient 18, after exiting a distal end of the sheath 21, and contact tissue of the heart. The distal end 22 comprises a position sensor 25 that enables the location and orientation of the distal end 22 to be tracked, and one or more temperature sensors 28 that measure the temperature at respective locations of the distal end 22. The temperature sensor(s) 28 is configured to provide a temperature signal which is indicative of a temperature of the myocardium 16 at a plurality of different times. The distal end 22 also comprises an electrode 30 which is configured to apply radiofrequency (RF) power to the myocardium 16 in the chamber so as to ablate the myocardium 16.

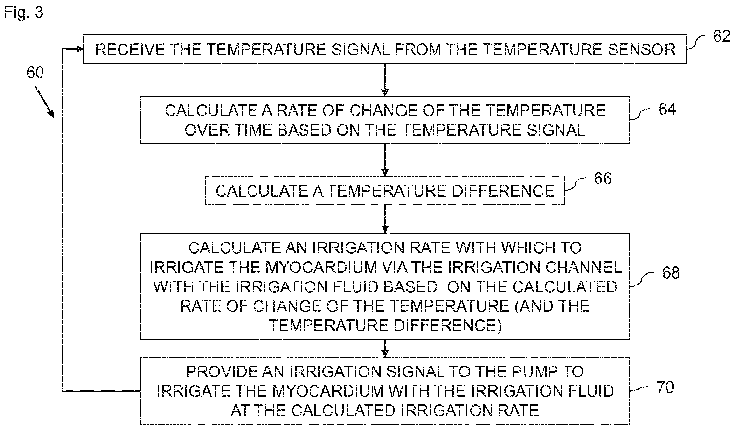

The apparatus 12 is controlled by a controller 46. The controller 46 is located in an operating console 48 of the apparatus 12. The controller 46 is described in more detail with reference to Fig. 3. The console 48 comprises controls 49 which are used by professional 14 to communicate with controller 46.

The controller 46 may include real-time noise reduction circuitry (not shown), typically configured as a field programmable gate array (FPGA), followed by an analog-to-digital (A/D) signal conversion integrated circuit (not shown). The controller 46 can pass the signals from the A/D circuit to modules described herein, and/or another controller 46 and/or can be programmed to perform at least one of the algorithms disclosed herein, the algorithms comprising steps described hereinbelow. The controller 46 may use the circuitry and the circuit mentioned above, as well as features of the modules referred to above, in order to perform the algorithms. The controller 46 and the modules operated by the controller 46 are herein termed processing circuitry. To implement the various procedures described herein, the controller 46 communicates with modules in a module bank 50. Modules in the module bank 50 are described below.

As stated above, in order to operate apparatus 12, controller 46 communicates with module bank 50. Thus, bank 50 comprises a tracking module 58 which receives and analyzes signals from the position sensor 25, and uses the signal analysis to generate a location and an orientation of the distal end 22. In some embodiments, the sensor 25 comprises one or more coils which provide the sensor signals in response to magnetic fields traversing the coils. In these embodiments, in addition to receiving and analyzing signals from the sensor 25, the tracking module 58 may also control magnetic radiators (not shown in the figures) which radiate the magnetic fields traversing sensor 25. The radiators are positioned in proximity to myocardium 16, and are configured to radiate alternating magnetic fields into a region in proximity to the myocardium 16.

Alternatively, or additionally, the tracking module 58 may measure impedances between electrode 30 and electrodes (not shown in the figures) on the surface of patient 18, and the controller 46 and the tracking module 58 may use the impedances to track the location and orientation of the distal end 22. The Carto® system produced by Biosense Webster, of 33 Technology Drive, Irvine, CA 92618 USA, uses such a magnetic tracking system and an impedance tracking system.

The operating console 48 includes an RF signal generator 55 configured to generate RF power to be applied by the electrode 30 at the distal end 22, and one or more returning electrodes (not shown in the figures) on the skin of the patient 18, to ablate the myocardium 16. The module bank 50 also comprises an ablation module 54 which controls the RF signal generator. The ablation module 54 may control the level of the power supplied by the RF signal generator 55 according to other factors, for example, a current temperature of the myocardium 16 as described in more detail below. In embodiments of the present invention, an ablation target power, which is a maximum power that may be injected into the patient's tissue by electrode 30 may be set by the medical professional 14. Typically, the ablation target power is set within an approximate range of 20W to 70W, although the ablation target power may be set outside this range. The ablation module 54 may also set parameters of the injected current, such as its frequency, the level of the power injected, and the duration of the power injection.

In some embodiments of the present invention, the apparatus 12 is configured to operate in one of two power modes. In a low-power mode, the ablation target power is set to be less than or equal to a preset power level. In a high-power mode, the ablation target power is set to be greater than the preset power level. By way of example, in the description herein the preset power level is assumed to be 35W. However, it will be understood that the preset power level, separating the two power modes, may be higher or lower than 35W.

The module bank 50 also comprises a temperature module 52 to analyze signals received from the temperature sensor(s) 28 in the distal end 22. From the analyzed signals, the controller 46 determines temperatures of the distal end 22, and uses the temperatures in the algorithms described below.

During the procedure performed by professional 14, the distal end 22 is supplied with irrigation fluid, typically saline solution, from a pump 24, and the pump 24 pumps the irrigation fluid into an irrigation channel 26 to the distal end 22 of the probe 20. The module bank 50 also comprises an irrigation module 56 that controls the rate of flow of irrigation fluid from the pump 24. Irrigation module 56 is under overall control of controller 46. The irrigation fluid is expelled through irrigation holes 80 in the distal end 22 to irrigate the myocardium 16 in order to maintain the temperature of the myocardium 16 as close as possible to a preset target temperature. Determination of the irrigation rate with which to pump the irrigation fluid is described in more detail with reference to Fig. 3.

In accordance with some embodiments the pump 24 is a variable rate pump, for example, pumping between 0 to 60 ml/min. In accordance with other embodiments of the present invention a variable irrigation rate may be provided using the method described in US Patent Application Publication 2018/0263689 in which the pump 24 is assumed to be able to operate in one of two modes: an idle mode, wherein the pump pumps the irrigation fluid at a slow rate, also herein termed an idle rate, and a full flow mode, wherein the pump pumps the fluid at a fast rate, also herein termed a full flow rate. Each of the rates may be preset before the pump is used in apparatus 12, and in one embodiment the idle rate may be set within a range of 0 - 6 mL/min, and the full rate may be set within a range of 6 - 60 mL/min. In some embodiments the flow rate from pump 24 may be continuously adjusted by using a PID (proportional integral derivative) algorithm to control the flow rate.

In order to operate the apparatus 12, the module bank 50 typically comprises modules other than those described above, such as a force module which acquires signals from a force sensor in the distal end 22 and which analyzes the signals to determine a force on the distal end 22. For simplicity, such other modules and their associated sensors are not illustrated in Fig. 1. All modules may comprise hardware as well as software elements.

In practice, some or all of the functions of the controller 46 may be combined in a single physical component or, alternatively, implemented using multiple physical components. These physical components may comprise hard-wired or programmable devices, or a combination of the two. In some embodiments, at least some of the functions of the processing circuitry may be carried out by a programmable processor under the control of suitable software. This software may be downloaded to a device in electronic form, over a network, for example. Alternatively, or additionally, the software may be stored in tangible, non-transitory computer-readable storage media, such as optical, magnetic, or electronic memory.

Reference is now made to Fig. 3, which is a flow chart 60 showing exemplary steps in a method of operation of the ablation apparatus 12 of Fig. 1 according to an embodiment of the present invention. Reference is also made to Figs. 1 and 2. The controller 46 (Fig. 1) is configured to receive (block 62) the temperature signal(s) from the temperature sensor(s) 28. The controller 46 is configured to calculate (block 64) a rate of change of the temperature over time based on the received temperature signal. The controller 46 is configured to calculate (block 66) a temperature difference, which is equal to a current temperature of the myocardium 16 measured by the temperature sensor(s) 28 less a preset target temperature. Therefore, the temperature difference is a positive value when the current temperature is greater than the preset target temperature, and a negative value when the current temperature is less than the preset target temperature. By way of example only, a suitable range for the preset target temperature is between 50°C and 60°C.

The controller 46 is configured to calculate (block 68) an irrigation rate with which to irrigate the myocardium 16 via the irrigation channel 26 with the irrigation fluid based on the calculated rate of change of the temperature. In accordance with some embodiments, the controller 46 is configured to calculate the irrigation rate based both on the calculated rate of change of the temperature and on the temperature difference. In accordance with some embodiments, the controller 46 is configured to calculate the irrigation rate based on a function that yields a higher irrigation rate based on a higher rate of change of temperature. The function may also be configured to yield a higher irrigation rate based on a higher value of the temperature difference.

The controller 46 may be configured to calculate the irrigation rate according to the function for all values of the rate of change of temperature and for all values of the temperature difference. In accordance with some embodiments, the controller 46 may be configured to calculate the irrigation rate according to the function when the current temperature is greater than the preset target temperature or at a second temperature value which is less than the preset target temperature or for one or more ranges of the temperature difference and/or one or more ranges of the rate of change of temperature. For example, pumping of the irrigation fluid may maintained at a low rate (e.g., the idle rate of the pump 24) when the current temperature is less than the preset target temperature and pumping of the irrigation fluid may be determined according to the function when the current temperature is greater than the preset target temperature. By way of another example, pumping of the irrigation fluid may maintained at a low rate (e.g., the idle rate) when the rate of change of temperature is negative (i.e., the temperature of the myocardium 16 is decreasing) and pumping of the irrigation fluid may be determined according to the function when the rate of change of temperature is positive (i.e., the temperature of the myocardium 16 is increasing).

An example function for calculating the irrigation rate for each cycle of a plurality of cycles now follows.

$$\text{New irrigation rate (flow)} = \text{currentFlow} + \text{deltaFlow(Temp)} + \text{deltaFlow(Power)} \quad \text{(Equation 1)},$$

where
currentFlow is the current irrigation rate,

$$\text{deltaFlow(Temp)} = At * \Delta T + Bt * \text{TempSlope} + Ct * \int\Delta T + Dt * \text{avg}(\Delta T),$$

and

$$\text{deltaFlow(Power)} = Ap * \Delta P + Bp * \text{PowerSlope} + Cp * \int\Delta P + Dp * \text{avg}(\Delta P),$$

$\Delta T$ is the difference between TargetTemp (the target temperature) and Temp (the sampled temperature, which could be an average of several sample cycles),
TempSlope is equal to the rate of change of the sampled temperature and could be computed from averaged samples,
$\int\Delta T$ is an integral of $\Delta T$ and the integral time range may vary,
$\text{avg}(\Delta T)$ is an average of $\Delta T$,
$At$ is a tuning parameter for $\Delta T$,
$Bt$ is a tuning parameter for TempSlope,
$Ct$ is a tuning parameter for $\int\Delta T$,
$Dt$ is a tuning parameter for $\text{avg}(\Delta T)$,
$\Delta P$ is the difference between TargetPower (the target power) and Power (sampled power, which could be an average of several sample cycles),
PowerSlope is the rate of change of the sampled power and could be computed from averaged samples,
$\int\Delta P$ is an integral of $\Delta P$ and the integral time range may vary,
$\text{avg}(\Delta P)$ is an average of $\Delta P$,
$Ap$ is a tuning parameter for $\Delta P$,
$Bp$ is a tuning parameter for PowerSlope,
$Cp$ is a tuning parameter for $\int\Delta P$,
$Dp$ is a tuning parameter for $\text{avg}(\Delta P)$.

The initial irrigation rate (flow) may be calculated as follows:

$$\text{Flow} = \text{FlowLow} + (\text{FlowHigh} - \text{FlowLow}) / (\text{PowerHigh} - \text{PowerLow}) * (\text{TargetPower} - \text{PowerLow}) \quad \text{(Equation 2)},$$

where

FlowLow is the lowest irrigation rate provided by the system,
FlowHigh is the highest irrigation rate provided by the system,
PowerLow is the lowest power provided by the system, and
PowerHigh is the highest power provided by the system.

Example ranges and values for the various parameter are given below. The example ranges and values given below for the integrals refer to an example upper limit for the integration with the lower limit of integration being zero seconds. The example ranges and values for Avg($\Delta$T) and Avg($\Delta$P) refer to an example sampling time-range for computing the respective averages. However, it should be noted that the values may be any suitable value even outside of the ranges given below.

| Parameter | Example range | Example value |
|---|---|---|
| $\int\Delta T$ | 0.5sec to 2sec | 1sec |
| Avg($\Delta$T) | 1sec to 5sec | 2sec |
| At | -0.9 to -0.1 | -0.5 |
| Bt | -0.9 to -0.1 | -0.3 |
| Ct | 0 to 0.1 | 0.015 |
| Dt | -0.9 to 0 | -0.05 |
| $\int\Delta P$ | 0.5sec to 2sec | 1sec |
| Avg($\Delta$P) | 1sec to 5sec | 2sec |
| Ap | 0.1 to 0.9 | 0.3 |
| Bp | 0.1 to 0.9 | 0.2 |
| Cp | -0.1 to 0 | 0 |
| Dp | 0 to 0.9 | 0 |

The above example ranges and values assume that $\Delta$T is equal to TargetTemp (the target temperature) less Temp (the sampled temperature) and $\Delta$P is TargetPower (the target power) less Power (the sampled power).

It should be noted that the parameters may be floating-point numbers and any of the parameters may be optionally averaged over a time period which may vary. The new irrigation rate could be a floating-point number with a limited range.

The controller is operative to provide (block 70) an irrigation signal to the pump 24 to irrigate the myocardium 16 with the irrigation fluid at the calculated irrigation rate. The steps of blocks 62-70 are repeated periodically (for example, in the range of every 1 millisecond to 1 second) in order to quickly react to changes in the rate of change of temperature and the temperature difference. The repetition frequency may depend on various implementation details such as speed of communication with the pump 24 and the reaction time of the pump 24 to change to a new irrigation rate.

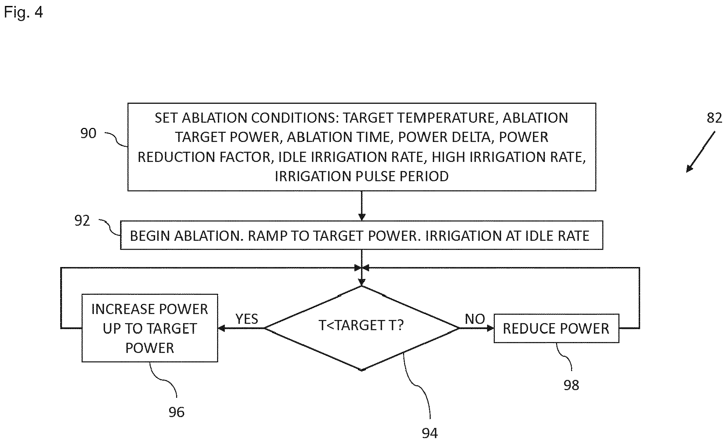

It should be noted that depending on the capabilities of the irrigation system and the heat supplied by the electrode 30, the irrigation fluid may be used to maintain the temperature of the myocardium 16 at the preset target temperature without having to reduce the RF power supplied to the electrode 30 below the target power. However, in addition to using the irrigation fluid to lower the temperature of the myocardium 16, the RF power supplied to the electrode 30 may also need to be (iteratively) reduced if the current temperature of the myocardium 16 is greater than a certain value, for example greater than the preset target temperature. Any suitable algorithm may be used to reduce the RF power. For example, US Patent Application Publication 2018/0263689 describes adjusting ablation power according to various factors based on a pump having two rates - an idle rate and a high rate. For the sake of completeness, some of the algorithms described in US Patent Application Publication 2018/0263689 are described below with reference to Figs. 4-9. Details of the algorithms may be changed to accommodate a given implementation.

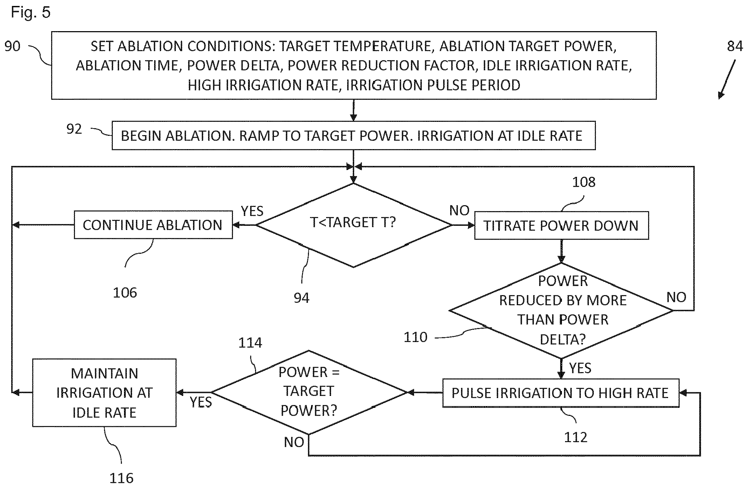

Reference is now made to Fig. 4, which is a first flowchart 82 of exemplary steps of an algorithm followed by controller 46 when apparatus 12 is operating in the low power mode described above, while professional 14 performs the ablation procedure referred to above, and to Fig. 5, which is a second flowchart 84 of exemplary steps of the algorithm followed by the controller 46, according to an embodiment of the present invention. As is described below, in the first flowchart, also referred to herein as flowchart 82, the controller 46 varies the power, and in the second flowchart, also referred to herein as flowchart 84, the controller 46 varies the irrigation rate. The controller 46 operates both flowcharts concurrently.

In the first flowchart (Fig. 4) in an initial step 90, typically performed prior to the actual ablation, the professional uses controls 49 to assign values to parameters used by the controller 46 in performing the algorithm.

Typical parameters set in the initial step comprise:

A target temperature, as measured as an average of sensors 28, which is an upper threshold temperature for performance of the ablation. In a disclosed embodiment the target temperature is set at 55°C, although the target temperature may typically be set in a range from 50°C to 60°C, or outside this range of values.

The ablation target power, which, as stated above, is a maximum power that may be injected into the patient's tissue by electrode 30. Ablation module 54 uses the ablation target power value to ensure that the injected power does not exceed this value. For the descriptions herein of the flowcharts of Fig. 4 and Fig. 5, the ablation target power is assumed to be set at 35 W, so that apparatus 12 is operating in its low power mode.

An ablation time, which is a maximum overall time period, used by ablation module 54, for which a single ablation is performed. In a disclosed embodiment the ablation time is set at 60 seconds (s).

A power delta, which is a change in power that the controller 46 checks in evaluating a condition in the algorithm. In a disclosed embodiment the power delta is set at 1W. A typical range for the power delta is 0.5W - 5W.

A power reduction factor, which is a reduction in power that the controller 46 implements when titrating the power to a lower value. In a disclosed embodiment the power reduction factor is set at 0.1 W. A typical range for the reduction factor is 0.05W - 0.2W.

An idle irrigation flow rate, which is the flow rate of pump 24 when the irrigation module 56 sets the pump to operate in its idle mode. A typical range for the idle irrigation flow rate is 1mL/min to 5mL/min, and in a disclosed embodiment the rate is set at 4mL/min.

A high irrigation flow rate, which is the flow rate of pump 24 when the irrigation module 56 sets the pump to operate in its full flow mode. A typical range for the high irrigation flow rate is 6mL/min to 60mL/min, and in a disclosed embodiment the rate is set at 15mL/min.

An irrigation pulse period, which is the period of time in which the irrigation module 56 pulses the pump to toggle from its idle mode, to the full flow mode, then return to the idle mode, or alternatively, to toggle from its full flow mode, to the idle mode, then return to the full flow mode. In a disclosed embodiment the irrigation pulse period is 0.5s, and the period may typically range between 0. Is and 2s.

Once the parameters have been set in step 90, control of the algorithm proceeds to a begin ablation step 92, wherein the controller 46 ramps the power dissipated by electrode 30 up to the target power level set in step 90. Depending whether the target power level sets the apparatus to operate in the low power mode or the high-power mode, the irrigation rate is set accordingly, i.e., for the low-power mode at the low irrigation rate, and for the high-power mode at the high irrigation rate. Since, as stated above, the target power level is set in step 90 at 35W, corresponding to the low power mode, then in step 92 the irrigation rate is set at the idle irrigation flow rate.

In a condition 94, the controller 46 uses temperature module 52 to check if the maximum temperature measured by any one of sensors 28 is lower than the target temperature set in step 90. Condition 94 iterates at a preset rate, which in an embodiment of the present invention is every 33 milli seconds (ms).

If condition 94 returns positive, i.e., if the temperature is less than the target temperature, then in an increase power step 96 controller 46 uses the ablation module 54 to increase the power, typically by the same value as the power reduction factor set in step 90, up to the target power.

If condition 94 returns negative, then in a decrease power step 98 controller 46 uses the ablation module 54 to decrease the power by the power reduction factor. Further details of the power decrease are described in flowchart 84 (Fig. 5).

In flowchart 84 the initial steps of the flowchart, steps 90, 92, and 94, are as described above with reference to flowchart 82 (Fig. 4). If in flowchart 84 condition 94 returns positive, i.e., the maximum temperature is less than the target temperature, then in a continuing ablation step 106 the controller 46 continues with the ablation, and control returns to condition 94.

If condition 94 returns negative, i.e., the maximum temperature is equal to or greater than the target temperature, then in a power titration step 108 the controller 46 uses ablation module 54 to titrate the power level down by the preset reduction factor set in step 90. Control then continues to a second condition 110.

In second condition 110, the controller 46 interrogates ablation module 54 to find the level of power being injected into electrode 80, and the controller 46 checks if the level has been reduced by more than the power delta set in step 90. If the second condition returns negative, i.e., the power has not been reduced from the target power value by the power delta, control returns to condition 94, which continues to iterate at its preset rate.

If second condition 110 returns positive, i.e., the power has been reduced from the target power value by more than the power delta, control of the algorithm continues to an irrigation pulse step 112. In step 112 irrigation module 56 configures pump 24 to transfer from its idle mode, i.e., pumping at the idle rate set in step 90, to its full flow mode wherein the pump pumps the irrigation fluid at its high rate set in step 90. The transfer to the full flow mode continues for the irrigation pulse period set in step 90, after which module 56 returns pump 24 to pumping at its idle rate.

At the conclusion of step 112, control continues to a third condition 114, wherein the controller 46 checks if the power set in flowchart 82 (Fig. 4), is equal to the target power.

If condition 114 returns positive, i.e., the power is equal to the target power, then in a further continuing ablation step 116 the controller 46 uses the irrigation module 56 to maintain the irrigation rate at the idle rate, and transfers control back to first condition 94.

If condition 114 returns negative, i.e., the power has not returned to the target power, then control returns to irrigation pulse step 112, so that the irrigation rate again pulses to a high rate.

Controller 46 continues implementing the steps of the two flowcharts 82, 84 concurrently for the ablation time set in step 90, after which the implementation ceases.

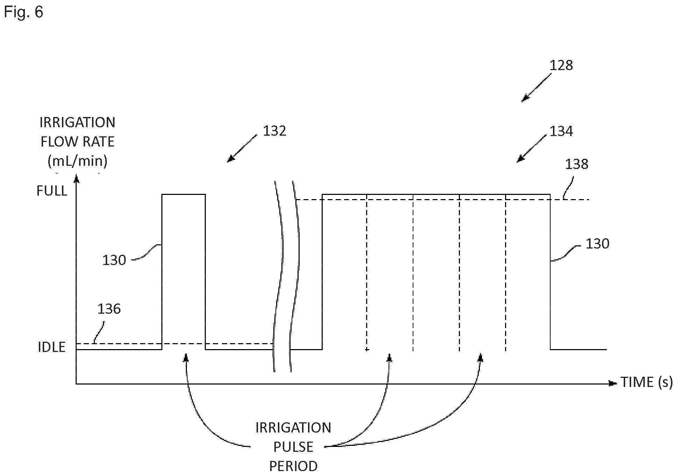

Reference is now made to Fig. 6, which illustrates graphically the operation of pump 24 while flowcharts 82, 84 are operative, according to an embodiment of the present invention. A graph 128 plots irrigation flow rate vs. time, and a solid line 130 of the graph illustrates the output flow rate of pump 24.

A section 132 of graph 128 illustrates the flow rate from pump 24, as solid line 130, as flowchart 84 proceeds to step 112, and then continues via condition 114, which returns positive, to step 116. In this case condition 114 is addressed only once, so that the flow rate from the pump begins at the idle rate, pulses for one irrigation pulse period to the high rate and then returns to the idle rate.

A section 134 of graph 128 illustrates the flow rate from pump 24, as solid line 130, as flowchart 84 proceeds to step 112, and then continues to condition 114, which returns negative, so returning to step 112. In this case condition 114 iterates, so that while the flow rate from the pump begins at the idle rate, the flow rate from the pump continues with multiple pulses, that present as effectively one long pulse, at the high rate.

As stated above solid line 130 illustrates the output of pump 24. However, the pulsatory output from the pump is smoothed, or averaged, by irrigation tubing 26, and the smoothed output is illustrated schematically by a broken line 136 for section 132, and a broken line 138 for section 134. The smoothed output is the irrigation flow rate at distal end 22.

For the irrigation pulse period of 0.5s of the disclosed embodiment referred to above, one pulse at a high rate of 15 mL/min, during an idle rate of 4 mL/min, typically increases the irrigation rate by between 50% and 100% of the idle rate, i.e., to an effective smoothed irrigation rate between 6 mL/min and 8 ml/min. A train of two or more pulses typically increases the effective irrigation rate to the high rate.

It will be understood that by varying the rate of pulsation of pump 24, and due to the smoothing effect of tubing 26, the irrigation flow rate at distal end 22 can be varied substantially continuously between the idle irrigation rate and the high irrigation rate.

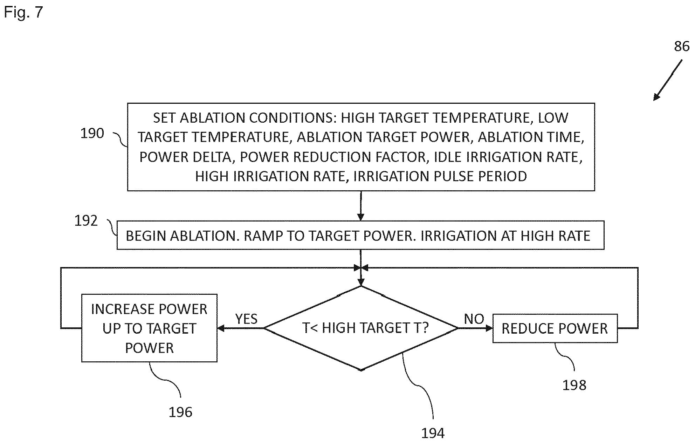

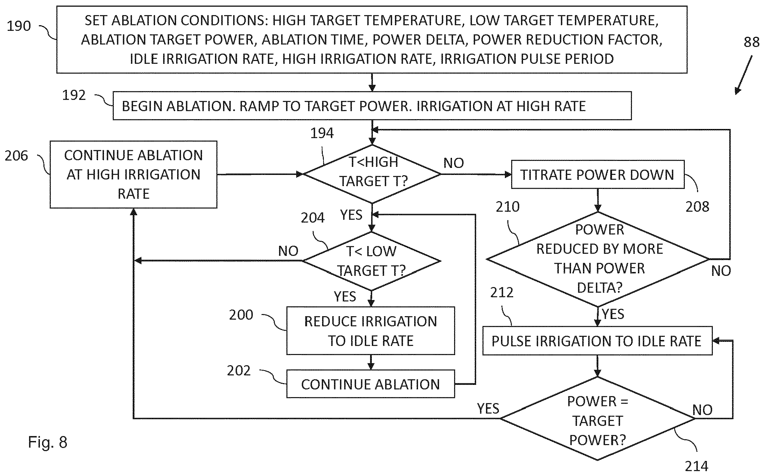

Reference is now made to Fig. 7, which is a first flowchart of steps of an alternative algorithm followed by controller 46 when apparatus 12 is operating in the high-power mode referred to above, while professional 14 performs the ablation procedure, and to Fig. 8, which is a second flowchart of steps of the alternative algorithm followed by the controller 46, according to an embodiment of the present invention. The flowchart of Fig. 7 is also referred to herein as flowchart 86, and the flowchart of Fig. 8 is also referred to herein as flowchart 88.

As for flowcharts 82 and 84 (Fig. 4 and Fig. 5), in flowchart 86 (Fig. 7) the controller 46 varies the power, and in flowchart 88 (Fig. 8) the controller 46 varies the irrigation rate; the controller 46 operates both flowcharts 86 and 88 concurrently.

An initial step 190 of flowchart 86 (Fig. 7) is substantially as described above for step 90, except that rather than setting one target temperature, a high target temperature and a low target temperature are set. The high target temperature is typically set to be in an approximate range of 40°C to 55°C, although values outside this range are possible. The low target temperature is typically set to be in an approximate range of 37°C to 50°C, although values outside this range are also possible. Regardless of the actual values of the high and low target temperatures, the low target temperature is set to be at least 1°C less than the high target temperature. In a disclosed embodiment the high target temperature is set at 50°C and the low target temperature is set at 45°C.

A condition 194 is substantially similar to condition 94, except that controller 46 uses temperature module 52 to check if the maximum temperature measured by any one of sensors 28 is lower than the high target temperature.

If condition 194 returns positive, i.e., if the temperature is less than the high target temperature, then in an increase power step 196 controller 46 uses the ablation module 54 to increase the power, typically by the same value as the power reduction factor set in step 190, up to the target power.

If condition 194 returns negative, then in a decrease power step 198 controller 46 uses the ablation module 54 to decrease the power by the power reduction factor. Further details of the power decrease are described in flowchart 88.

In flowchart 88 (Fig. 8) the initial steps of the flowchart, steps 190, 192, and 194, are as described above with reference to flowchart 86. If in flowchart 88 condition 194 returns positive, i.e., the maximum temperature is less than the high target temperature, then control transfers to a further condition 204, where the controller 46 checks if the maximum temperature is less than the low target temperature. Condition 204 typically iterates at the same preset rate as condition 194.

If condition 204 returns negative, so that the maximum temperature is between the low and high target temperatures, then control transfers to a continuing ablation step 206, wherein ablation is continued at the high irrigation rate set initially, and control returns to condition 194.

If condition 204 returns positive, so that the maximum temperature is below the low target temperature, then control transfers to a reduce irrigation step 200, where the controller 46 reduces the high irrigation rate set initially to the idle irrigation rate. Ablation continues at the idle irrigation rate in a continuing ablation step 202 and control transfers back to iterating condition 204.

The path of condition 204, step 200, and step 202 illustrates that while the maximum temperature is below the low target temperature, the controller 46 maintains the irrigation at its low idle rate.

Returning to condition 194, if the condition returns negative, i.e., the maximum temperature is equal to or greater than the high target temperature, then in a power titration step 208 the controller 46 titrates the power down, substantially as described in power titration step 108. Control then continues to a power reduction condition 210.

Condition 210 is substantially as described for condition 110, i.e., the controller 46 interrogates ablation module 54 to check if the power level has been reduced by more than the power delta set in step 190. If condition 210 returns negative, i.e., the power has not been reduced from the target power value by the power delta, control returns to condition 194, which continues to iterate at its preset rate.

If condition 210 returns positive, i.e., the power has been reduced from the target power value by more than the power delta, control of the algorithm continues to an irrigation pulse step 212. In step 212 irrigation module 56 configures pump 24 to transfer from its full flow mode, i.e., pumping at the high rate set in step 190, to its idle mode wherein the pump pumps the irrigation fluid at its low rate set in step 190. The transfer to the idle mode continues for the irrigation pulse period set in step 190, after which module 56 returns pump 24 to pumping at its full rate.

At the conclusion of step 212, control continues to a power check condition 214, wherein the controller 46 checks if the power set in flowchart 86 (Fig. 7), is equal to the target power.

If condition 214 returns positive, i.e., the power is equal to the target power, then control continues at continuing ablation step 206, where the irrigation module 56 maintains the irrigation rate at the full rate, and transfers control back to condition 194.

If condition 214 returns negative, i.e., the power has not returned to the target power, then control returns to irrigation pulse step 212, so that the irrigation rate again pulses to a low rate.

Controller 46 continues implementing the steps of the two flowcharts 86, 88 concurrently for the ablation time set in step 190, after which the implementation ceases.

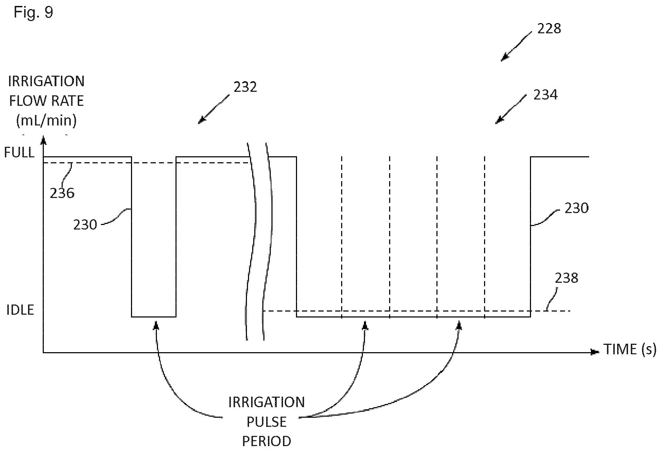

Reference is now made to Fig. 9, which illustrates graphically the operation of pump 24 while flowcharts 86, 88 are operative, according to an embodiment of the present invention. A graph 228 plots irrigation flow rate vs. time, and a solid line 230 of the graph illustrates the output flow rate of pump 24.

A section 232 of graph 228 illustrates the flow rate from pump 24, as solid line 230, as flowchart 88 proceeds to step 212, and then continues via condition 214, which returns positive, to step 206. In this case condition 214 is addressed only once, so that the flow rate from the pump begins at the full rate, pulses for one irrigation pulse period to the low rate and then returns to the idle rate.

A section 234 of graph 228 illustrates the flow rate from pump 24, as solid line 230, as flowchart 88 proceeds to step 212, and then continues to condition 214, which returns negative, so returning to step 212. In this case condition 214 iterates, so that while the flow rate from the pump begins at the high rate, the flow rate from the pump continues with multiple pulses, that present as effectively one long pulse, at the low rate.

As stated above solid line 230 illustrates the output of pump 24. However, the pulsatory output from the pump 24 is smoothed, or averaged, by irrigation tubing 26, and the smoothed output is illustrated schematically by a broken line 236 for section 232, and a broken line 238 for section 234. The smoothed output is the irrigation flow rate at distal end 22.

The smoothing is generally similar to that described above with respect to Fig. 6. Thus, for an irrigation pulse period of 0.5s, a single pulse at an idle rate of 4 mL/min, during a high rate of 15 mL/min, typically reduces the irrigation rate by approximately 50% of the high rate, i.e., to approximately 8 mL/min. A train of two or more pulses typically reduces the effective irrigation rate to the idle rate.

Various features of the invention which are, for clarity, described in the contexts of separate embodiments may also be provided in combination in a single embodiment. Conversely, various features of the invention which are, for brevity, described in the context of a single embodiment may also be provided separately or in any suitable sub-combination.

The embodiments described above are cited by way of example, and the present invention is not limited by what has been particularly shown and described hereinabove. Rather the scope of the invention includes both combinations and subcombinations of the various features described hereinabove, as well as variations and modifications thereof which would occur to persons skilled in the art upon reading the foregoing description and which are not disclosed in the prior art.

**Claims**

1. An irrigated ablation system, comprising:

a probe being configured to be inserted into a chamber of a heart, the probe including:

an electrode configured to apply radiofrequency (RF) power to a myocardium in the chamber so as to ablate the myocardium;

a temperature sensor configured to provide a temperature signal which is indicative of a temperature of the myocardium at a plurality of different times; and

an irrigation channel through which to irrigate the myocardium;

a pump to pump an irrigation fluid into the irrigation channel;

an RF signal generator configured to generate the RF power to be applied by the electrode to ablate the myocardium; and

a controller configured to:

receive the temperature signal from the temperature sensor;

calculate a rate of change of the temperature over time based on the temperature signal;

calculate an irrigation rate with which to irrigate the myocardium via the irrigation channel with the irrigation fluid based at least on the calculated rate of change of the temperature; and

provide an irrigation signal to the pump to irrigate the myocardium with the irrigation fluid at the calculated irrigation rate.

2. The system according to claim 1, wherein the controller is configured to calculate the irrigation rate based both on the calculated rate of change of the temperature and on a temperature difference, which is equal to a current temperature measured by the temperature sensor less a preset target temperature.

3. The system according to claim 2, wherein the controller is configured to calculate the irrigation rate based on a function that yields a higher irrigation rate based on a higher rate of change of temperature.

4. The system according to claim 3, wherein the function is configured to yield a higher irrigation rate based on a higher value of the temperature difference.

5. The system according to claim 4, wherein the controller is configured to calculate the irrigation rate based on: the calculated rate of change of the temperature; the temperature difference; a rate of change of the RF power; and a RF power difference, which is equal to a difference between a current value of the RF power and a preset target RF power.

6. An irrigated ablation method comprising:

generating radiofrequency (RF) power to be applied by an electrode of a probe to ablate a myocardium in a chamber of a heart;

applying the RF power to the myocardium so as to ablate the myocardium;

providing a temperature signal which is indicative of a temperature of the myocardium at a plurality of different times;

pumping an irrigation fluid into an irrigation channel through which to irrigate the myocardium;

receiving the temperature signal;

calculating a rate of change of the temperature over time based on the temperature signal;

calculating an irrigation rate with which to irrigate the myocardium via the irrigation channel with the irrigation fluid based at least on the calculated rate of change of the temperature; and

providing an irrigation signal to a pump to irrigate the myocardium with the irrigation fluid at the calculated irrigation rate.

7. The method according to claim 6, wherein the calculating the irrigation rate includes calculating the irrigation rate based both on the calculated rate of change of the temperature and on a temperature difference, which is equal to a current temperature measured by the temperature sensor less a preset target temperature.

8. The method according to claim 7, wherein the irrigation rate is calculated based on a function that yields a higher irrigation rate based on a higher rate of change of temperature.

9. The method according to claim 8, wherein the function is configured to yield a higher irrigation rate based on a higher value of the temperature difference.

**10.** The method according to claim 9, wherein the calculating the irrigation rate includes calculating the irrigation rate based on: the calculated rate of change of the temperature; the temperature difference; a rate of change of the RF power; and a RF power difference, which is equal to a difference between a current value of the RF power and a preset target RF power.

**11.** A software product, comprising a non-transient computer-readable medium in which program instructions are stored, which instructions, when read by a central processing unit (CPU), cause the CPU to:

receive a temperature signal which is indicative of a temperature of a myocardium of a chamber of a heart at a plurality of different times;
calculate a rate of change of the temperature over time based on the temperature signal;
calculate an irrigation rate with which to irrigate the myocardium via an irrigation channel with an irrigation fluid based at least on the calculated rate of change of the temperature; and
provide an irrigation signal to a pump to irrigate the myocardium with the irrigation fluid at the calculated irrigation rate.

Fig. 1

MODULE BANK

TRACKING MODULE — 58

IRRIGATION MODULE — 56

TEMPERATURE MODULE — 52

ABLATION MODULE — 54

PROCESSOR — 46

Fig. 2

Fig. 3

RECEIVE THE TEMPERATURE SIGNAL FROM THE TEMPERATURE SENSOR — 62

60

CALCULATE A RATE OF CHANGE OF THE TEMPERATURE
OVER TIME BASED ON THE TEMPERATURE SIGNAL — 64

CALCULATE A TEMPERATURE DIFFERENCE — 66

CALCULATE AN IRRIGATION RATE WITH WHICH TO
IRRIGATE THE MYOCARDIUM VIA THE IRRIGATION CHANNEL
WITH THE IRRIGATION FLUID BASED ON THE CALCULATED — 68
RATE OF CHANGE OF THE TEMPERATURE (AND THE
TEMPERATURE DIFFERENCE)

PROVIDE AN IRRIGATION SIGNAL TO THE PUMP TO
IRRIGATE THE MYOCARDIUM WITH THE IRRIGATION FLUID — 70
AT THE CALCULATED IRRIGATION RATE

EP 3 659 536 A1

16

Fig. 4

90 — SET ABLATION CONDITIONS: TARGET TEMPERATURE, ABLATION TARGET POWER, ABLATION TIME, POWER DELTA, POWER REDUCTION FACTOR, IDLE IRRIGATION RATE, HIGH IRRIGATION RATE, IRRIGATION PULSE PERIOD

82

92 — BEGIN ABLATION. RAMP TO TARGET POWER. IRRIGATION AT IDLE RATE

INCREASE POWER UP TO TARGET POWER

YES

T<TARGET T?

NO

REDUCE POWER

98

96

94

EP 3 659 536 A1

17

Fig. 5

90 — SET ABLATION CONDITIONS: TARGET TEMPERATURE, ABLATION TARGET POWER, ABLATION TIME, POWER DELTA, POWER REDUCTION FACTOR, IDLE IRRIGATION RATE, HIGH IRRIGATION RATE, IRRIGATION PULSE PERIOD

84

92 — BEGIN ABLATION. RAMP TO TARGET POWER. IRRIGATION AT IDLE RATE

108

CONTINUE ABLATION ←YES— T<TARGET T? —NO→ TITRATE POWER DOWN

106

94

POWER REDUCED BY MORE THAN POWER DELTA? —NO

110

114

MAINTAIN IRRIGATION AT IDLE RATE ←YES— POWER = TARGET POWER? ← PULSE IRRIGATION TO HIGH RATE ← YES

NO

112

116

EP 3 659 536 A1

18

Fig. 6

Fig. 7

86

190 — SET ABLATION CONDITIONS: HIGH TARGET TEMPERATURE, LOW TARGET TEMPERATURE, ABLATION TARGET POWER, ABLATION TIME, POWER DELTA, POWER REDUCTION FACTOR, IDLE IRRIGATION RATE, HIGH IRRIGATION RATE, IRRIGATION PULSE PERIOD

192 — BEGIN ABLATION. RAMP TO TARGET POWER. IRRIGATION AT HIGH RATE

INCREASE POWER UP TO TARGET POWER — 196

YES ← T< HIGH TARGET T? → NO → REDUCE POWER — 198

194

190 — SET ABLATION CONDITIONS: HIGH TARGET TEMPERATURE, LOW TARGET TEMPERATURE, ABLATION TARGET POWER, ABLATION TIME, POWER DELTA, POWER REDUCTION FACTOR, IDLE IRRIGATION RATE, HIGH IRRIGATION RATE, IRRIGATION PULSE PERIOD

88

192 — BEGIN ABLATION. RAMP TO TARGET POWER. IRRIGATION AT HIGH RATE

194

206 — CONTINUE ABLATION AT HIGH IRRIGATION RATE

T<HIGH TARGET T?

NO → TITRATE POWER DOWN

210

208

YES

204

NO

T< LOW TARGET T?

POWER REDUCED BY MORE THAN POWER DELTA?

NO

212

YES

200 — REDUCE IRRIGATION TO IDLE RATE

YES

PULSE IRRIGATION TO IDLE RATE

202 — CONTINUE ABLATION

YES

POWER = TARGET POWER?

NO

214

Fig. 8

Fig. 9

EP 3 659 536 A1

Europäisches
Patentamt

European
Patent Office

Office européen
des brevets

## EUROPEAN SEARCH REPORT

Application Number

EP 19 20 9947

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/312025 A1 (HARLEV DORON [US] ET AL) 2 November 2017 (2017-11-02) * paragraphs [0243] - [0280] * | 1-11 | INV. A61B18/12 A61B18/14 |
| A | WO 2018/200865 A1 (EPIX THERAPEUTICS INC [US]) 1 November 2018 (2018-11-01) * paragraphs [0001], [0186], [0365] - [0370] * | 1-11 | ADD. A61B18/00 |
| A,D | US 2018/263689 A1 (GOVARI ASSAF [IL] ET AL) 20 September 2018 (2018-09-20) * paragraphs [0030] - [0096] * | 1-11 | |
| A | EP 3 375 397 A1 (BIOSENSE WEBSTER ISRAEL LTD [IL]) 19 September 2018 (2018-09-19) * claims 1-5 * | 1-11 | |

TECHNICAL FIELDS
SEARCHED      (IPC)

A61B

The present search report has been drawn up for all claims

| Place of search | Date of completion of the search | Examiner |
|---|---|---|
| Munich | 24 April 2020 | Aronsson, Fredrik |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 19 20 9947

This annex lists the patent family members relating to the patent documents cited in the above-mentioned European search report.
The members are as contained in the European Patent Office EDP file on
The European Patent Office is in no way liable for these particulars which are merely given for the purpose of information.

24-04-2020

| Patent document cited in search report | Publication date | Patent family member(s) | Publication date |
|---|---|---|---|
| US 2017312025 A1 | 02-11-2017 | CN 109414286 A | 01-03-2019 |
| | | EP 3451954 A1 | 13-03-2019 |
| | | EP 3451961 A1 | 13-03-2019 |
| | | EP 3451962 A2 | 13-03-2019 |
| | | EP 3451963 A2 | 13-03-2019 |
| | | JP 2019515755 A | 13-06-2019 |
| | | JP 2019515756 A | 13-06-2019 |
| | | US 2017312007 A1 | 02-11-2017 |
| | | US 2017312008 A1 | 02-11-2017 |
| | | US 2017312012 A1 | 02-11-2017 |
| | | US 2017312020 A1 | 02-11-2017 |
| | | US 2017312023 A1 | 02-11-2017 |
| | | US 2017312024 A1 | 02-11-2017 |
| | | US 2017312025 A1 | 02-11-2017 |
| | | US 2017312026 A1 | 02-11-2017 |
| | | US 2017312027 A1 | 02-11-2017 |
| | | US 2017312028 A1 | 02-11-2017 |
| | | US 2017312420 A1 | 02-11-2017 |
| | | US 2019076190 A1 | 14-03-2019 |
| | | WO 2017192477 A1 | 09-11-2017 |
| | | WO 2017192480 A2 | 09-11-2017 |
| | | WO 2017192495 A1 | 09-11-2017 |
| | | WO 2017192510 A2 | 09-11-2017 |
| | | WO 2017192542 A2 | 09-11-2017 |
| WO 2018200865 A1 | 01-11-2018 | CN 110809448 A | 18-02-2020 |
| | | EP 3614946 A1 | 04-03-2020 |
| | | US 2019038348 A1 | 07-02-2019 |
| | | US 2019038349 A1 | 07-02-2019 |
| | | WO 2018200865 A1 | 01-11-2018 |
| US 2018263689 A1 | 20-09-2018 | AU 2018201563 A1 | 04-10-2018 |
| | | CA 2998013 A1 | 14-09-2018 |
| | | CN 108567481 A | 25-09-2018 |
| | | EP 3375396 A1 | 19-09-2018 |
| | | JP 2018149301 A | 27-09-2018 |
| | | US 2018263689 A1 | 20-09-2018 |
| EP 3375397 A1 | 19-09-2018 | AU 2018201564 A1 | 04-10-2018 |
| | | CA 2998011 A1 | 14-09-2018 |
| | | CN 108720838 A | 02-11-2018 |
| | | EP 3375397 A1 | 19-09-2018 |
| | | JP 2018149300 A | 27-09-2018 |
| | | US 2018263690 A1 | 20-09-2018 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 19625518, Govari
- US 20110022041 A, Frank
- US 5304214 A, DeFord

- US 20080275440 A, Kratoska
- US 20110077639 A, Brannan
- US 20180263689